(19) Europäisches Patentamt
European Patent Office
Office européen des brevets

(11) Veröffentlichungsnummer: **0 116 827 B1**

(12) # EUROPÄISCHE PATENTSCHRIFT

(45) Veröffentlichungstag der Patentschrift: **10.04.91**

(51) Int. Cl.[5]: **C07D 277/82**, C07D 277/64

(21) Anmeldenummer: **84100133.2**

(22) Anmeldetag: **09.01.84**

Die Akte enthält technische Angaben, die nach dem Eingang der Anmeldung eingereicht wurden und die nicht in dieser Patentschrift enthalten sind.

(54) **Verfahren zur Herstellung von Benzthiazolen.**

(30) Priorität: **21.01.83 DE 3301869**
**18.10.83 DE 3337859**

(43) Veröffentlichungstag der Anmeldung:
**29.08.84 Patentblatt 84/35**

(45) Bekanntmachung des Hinweises auf die Patenterteilung:
**10.04.91 Patentblatt 91/15**

(84) Benannte Vertragsstaaten:
**CH DE FR GB LI**

(56) Entgegenhaltungen:
**EP-A- 0 069 445**
**GB-A- 410 088**
**US-A- 2 575 614**

(73) Patentinhaber: **BAYER AG**
**W-5090 Leverkusen 1 Bayerwerk(DE)**

(72) Erfinder: **Hamprecht, Rainer, Dr.**
**Im Kerberich 25**
**W-5068 Odenthal(DE)**

EP 0 116 827 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Gegenstand der Erfindung ist ein Verfahren zur Herstellung von Benzthiazolderivaten der Formel

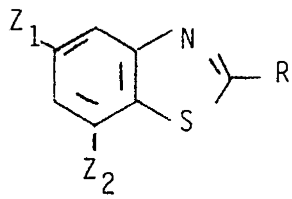

worin

| | |
|---|---|
| R | für H, $NH_2$ oder $R_1$ steht, wobei |
| $R_1$ | $C_1$-$C_6$-Alkyl oder Phenyl , gegebenenfalls substituiert durch $C_1$-$C_6$-Alkyl , $NO_2$, $CF_3$, -$SO_2$-$C_1$-$C_6$-Alkyl , Halogen oder CN bedeutet, |
| $Z_1$ und $Z_2$ | Wasserstoff, Cl , Br, $NO_2$, $CF_3$, CN, -CO-Alkyl, -$SO_2$-Alkyl, -$SO_2$-Phenyl, -$CO_2$-Alkyl, -CON-(Alkyl)$_2$ oder -$SO_2$N(Alkyl)$_2$ bedeuten, wobei $Z_1$ und $Z_2$ nicht gleichzeitig für Wasserstoff stehen, die Alkylreste 1-6 C-Atome aufweisen und der Phenylrest durch Cl, Br, $NO_2$ oder $C_1$-$C_4$-Alkoxy substituiert sein kann, |

dadurch gekennzeichnet, daß man Verbindungen der Formel

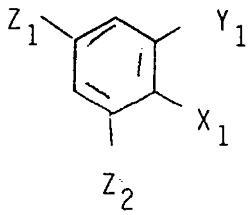

(II)

worin

$X_1$ Chlor, Brom, SH, SCN oder einen Rest der Formel (III)

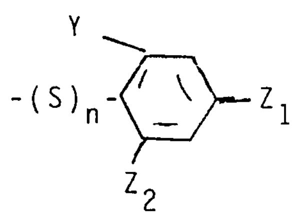

(III)

n = 1 oder 2,

Y $NO_2$ oder $NH_2$,

$Y_1$ $NO_2$ und - falls $X_1$ = SH oder einen Rest der Formel (III ) mit n = 2 - auch $NH_2$ bedeuten,

a) mit einer Verbindung, die in einer ihrer möglichen tautomeren Formen der Formel

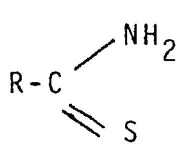

(IV)

entspricht,

oder

b) mit einem Rhodanid der Formel $NH_4{}^+SCN^-$ oder $R_1$-$NH_3{}^+SCN^-$
oder

c) mit einem Gemisch aus Nitrilen der Formel $R_1$-CN oder $NH_2CN$ und $H_2S$,
umsetzt.

Dabei soll der Schwefelwasserstoff zweckmäßigerweise im Überschuß vorliegen.

Während die Verbindungen der Formel (IV) mit Verbindungen der Formel II, worin Y = $NH_2$ bedeutet, auch äquimolar zum Einsatz gelangen können, müssen sie gegenüber den Verbindungen (II) mit Y = $NO_2$ im Überschuß eingesetzt werden. Im allgemeinen reicht ein kleiner Überschuß von 5 bis 10% eines Äquivalents. Vorzugsweise arbeitet man aber mit 2 bis 10, besonders bevorzugt mit 3 bis 6 Äquivalenten an (IV) bezogen auf (II).

Ganz besonders bevorzugt sind Verbindungen der Formel (II), worin

$Z_1$ $CF_3$, $SO_2CH_3$, $SO_2C_2H_5$, -$SO_2C_6H_5$ und vor allem $NO_2$,
$Z_2$ $CF_3$, $NO_2$ oder vor allem H,
$X_1$ Cl und
$Y_1$ $NO_2$ bedeuten.

Die Umsetzung von (II) mit (IV) kann grundsätzlich ohne je Verdünnungsmittel, vorteilhafterweise in einem größeren Überschuß von geschmolzenem (IV) durchgeführt werden. Es empfiehlt sich jedoch in den meisten Fällen, die Umsetzung in Gegenwart eines unter den Reaktionsbedingungen indifferenten Lösungsmittels vorzunehmen, da die Reaktion mehr oder weniger stark exotherm ist und die entstehende Wärme so besser abgeführt werden kann.

Geeignete Lösungsmittel sind vor allem solche organischer Natur wie höher siedende Alkohole (insbesondere Propanol, Butanole und Pentanole), Glykolether (z.B. Methyl-, Ethyl- und Butylglykol, Diethylenglykoldimethylether), cyclische Amide (z.B. N-Methylpyrrolidon und Caprolactam) Ketone wie z.B. Diethylketon sowie vor allem Tetrahydrothiophen-1,1-dioxid.

Auch Wasser und wäßrige Zweiphasensysteme (z.B. Toluol/ Wasser) ggf. in Gegenwart von Phasentransferkatalysatoren kommen als Lösungsmittel in Betracht.

Dagegen sind Lösungsmittel mit einer Carbonsäurefunktion weniger geeignet, da sie unter bestimmten Bedingungen an der Reaktion teilnehmen.

Im allgemeinen wird die Umsetzung bei Temperaturen von 60 bis 250° C, vorzugsweise 70 bis 200° C und besonders bevorzugt bei 70 bis 150° C, durchgeführt.

Bei der praktischen Durchführung des neuen Verfahrens geht man zweckmäßigerweise so vor, daß man in eine Suspension oder Lösung der Verbindungen (IV) bzw. der sie bildenden Systeme die Verbindungen (II) in der Weise einträgt, daß die in Vorversuchen als geeignet bestimmte Reaktionstemperatur nicht wesentlich überschritten wird. Gegebenenfalls kann dabei das Reaktionsgefäß gekühlt werden.

Ein Zusatz von Basen (z.B. tert.-Amine oder N-Heterocyclen) ist häufig von Vorteil.

Das Ende der Reaktion bzw. die Vollständigkeit der Umsetzung wird am besten dünnschichtchromatographisch ermittelt.

Die Verfahrensprodukte, die zum großen Teil bekannt sind, sind wertvolle Ausgangsmaterialien für diverse technische Produkte. Hervorzuheben ist ihre Eignung zur Herstellung von Azofarbstoffen (DE-PS 639 727), Sensibilisatoren (DE-PS 710 748), Metallkomplexfarbstoffe (DE-OS 2 848 622), Herbiziden (US-PS 2 756 135) und Polymethinen mit breitem Applikationsspektrum (vgl. Houben-Weyl, "Methoden der Organischen Chemie", 4. Auflage, Band V/1a, Seiten 231 ff).

Das neue Verfahren zeichnet sich gegenüber der bekannten Hugershoff-Synthese (R.C. Elderfield, "Heterocyclic Compounds", Vol. 5, 581 ff, John Wiley & Sons, New York 1957 sowie DE-OS 2 631 163) durch eine besonders einfache Verfahrensweise aus, da aus technisch leicht zugänglichen Ausgangsmaterialien wie z.B. o-Chlor-nitro-benzolderivaten in einer einstufigen Reaktion 2-Amino-benzthiazole gebildet werden, während nach Hugershoff aus Anilinderivaten erst die entsprechenden Phenylthioharnstoffe dargestellt werden müssen, die dann mittels Oxydationsmitteln in einem zweiten Verfahrensschritt zu 2-Aminobenzthiazolen cyclisiert werden.

Ein weiterer Nachteil der Hugershoff-Synthese ist darin zu sehen, daß im Falle meta-substituierter Aniline kein einheitliches Produkt, sondern Gemische von 5- und 7-substituierten 2-Aminobenzthiazolen entstehen (vgl. DOS 2 602 173 = US 4 052 379). Das Hugershoff-Verfahren ist weiterhin dadurch eingeschränkt, daß insbesondere stark elektronegativ substituierte Phenylthioharnstoffe schlecht zugänglich sind und z.T. Umwege über die entsprechenden Acyl-phenylthioharnstoffe (DOS 2 602 173) in Kauf genommen werden müssen.

Ähnliche Nachteile gelten für die oxydative Rhodanierung von Anilinen (vgl. Die Pharmazie, 32 , 195, (1977)).

Schließlich ist aus EP-A 0 069 445 ein Verfahren zur Herstellung von 5-gliedrigen Heterocyclen bekannt, wobei unter anderem in Beispiel 16 der Tabelle 1 die Umsetzung von o-Aminothiophenol mit $NH_4SCN$ erwähnt wird. Gemäß Seite 6, Zeile 26 soll dabei 2-Mercaptobenzthiazol entstehen.

Beispiel 1

Darstellung von 2-Amino-5-nitrobenzothiazol

Eine Suspension von 10,13 g 2,4-Dinitro-chlorbenzol und 15,2 g Thioharnstoff in 50 ml Sulfolan (Tetrahydrothiophen-1,1-dioxid) wird 12 Stunden bei 110 bis 120 °C gerührt. Nach Abkühlen wird mit 800 ml Wasser verrührt, abgesaugt und mit Wasser nachgewaschen. Nach Trocknen erhält man 11,2 g eines gelben Pulvers, das nach HPLC-Analyse (High pressure liquid chromatography) 69,5 % 2-Amino-5-nitrobenzthiazol (entsprechend 80 % der Theorie) enthält. Umkristallisieren aus Dimethylformamid hebt den Schmelzpunkt auf 307 °C (Zers.) an (Zhur. Obschei Khim. 30 , 1363-6 (1960) gibt Fp. 308-309 °C Zers. an).

Beispiel 2

Darstellung von 2-Amino-5-nitrobenzthiazol

Eine Lösung von 10,13 g 2,4-Dinitrochlorbenzol und 15,2g Thioharnstoff in 50 ml Pyridin wird 3 Stunden unter Rühren am Rückflußkühler gekocht. Nach Erkalten wird mit 500 ml Wasser verrührt, abgesaugt, mit Wasser gewaschen und getrocknet. Man erhält 11,6 g mit einer Reinheit von 66 % nach HPLC-Analyse entsprechend einer Ausbeute von 78,5 % der Theorie 2-Amino-5-nitrobenzthiazol.

Beispiel 3

Darstellung von 2-Amino-5-nitrobenzthiazol

Eine Suspension von 5,06 g 2,4-Dinitrochlorbenzol und 7,6 g Ammoniumrhodanid in 25 ml Sulfolan wird unter Rühren auf 150 °C erhitzt und 1,5 Stunden bei dieser Temperatur gehalten. Man trägt auf 300 ml Wasser aus, saugt ab und wäscht mit Wasser. Nach Trocknen erhält man 6,57 g rohes 2-Amino-5-nitrobenzthiazol. Die Identität wurde durch DC-Vergleich (DC = Dünnschichtchromatographie) mit authentischem Material gesichert.

Beispiel 4

Darstellung von 2-Amino-5-nitrobenzthiazol

Zu einer Lösung von 7,6 g Thioharnstoff in 50 ml Dimethylformamid fügt man bei 150 °C innerhalb von 15 Minuten 11,71 g 2,4-Dinitrophenylrhodanid. Man rührt weitere 2 Stunden bei 150 °C, trägt auf 500 ml Wasser aus, saugt ab und wäscht mit Wasser. Man erhält 11 g rohes 2-Amino-5-nitrobenzthiazol, dessen Identität durch DC-Vergleich mit authentischem Material gesichert wurde.

Beispiel 5

Darstellung von 2-Amino-5-nitrobenzthiazol

Eine Lösung von 10 g 2,4-Dinitrothiophenol und 7,6 g Thioharnstoff in 50 ml Dimethylformamid wird 3 Stunden bei 120 °C gerührt. Man verdünnt mit 300 ml Wasser, saugt ab und wäscht mit Wasser. Man erhält 7,3 g rohes 2-Amino-5-nitrobenzthiazol, dessen Identität durch DC-Vergleich mit authentischem Material gesichert wurde.

Beispiel 6

Darstellung von 2-Amino-5-nitrobenzthiazol

Eine Lösung von 5,97 g 2,4,2',4'-Tetranitro-diphenyl-disulfid und 4,56 g Thioharnstoff in 40 ml Dimethylformamid wird 3 Stunden bei 120 °C gerührt. Man trägt auf 300 ml Wasser aus, saugt ab und wäscht mit Wasser. Man erhält 4,5 g rohes 2-Amino-5-nitrobenzthiazol vom Fp. 290-300 °C (Zers.).

Beispiel 7

Darstellung von 2-Amino-5-nitrobenzthiazol

Zu einer Lösung von 7,6 g Thioharnstoff in 50 ml Dimethylformamid fügt man bei 120 °C in 15 Minuten 9,15 g 2,4,2',4'-Tetranitrodiphenylsulfid und rührt 2 Stunden bei dieser Temperatur. Man verdünnt mit 300 ml Wasser, saugt ab und wäscht mit Wasser. Man erhält 7,6 g rohes 2-Amino-5-nitrobenzthiazol vom Fp. 290-300 °C (Zers.).

Beispiel 8

Darstellung von 2-Amino-5-nitrobenzthiazol

Eine Lösung von 8,63 g 2-Chlor-5-nitroanilin und 9,9 g Natriumsulfid-trihydrat in 80 ml Butylglykol wurde 30 Minuten bei 80 °C gerührt. Nach Zusatz von 4,16 ml konz. Schwefelsäure wurde das 2-Amino-4-nitrothiophenol mit 7,6 g Thioharnstoff versetzt und 3 Stunden bei 120 °C gerührt. Das Reaktionsgemisch wurde auf 400 ml Wasser und 50 ml Methanol ausgetragen, abgesaugt und mit Wasser gewaschen. Man erhielt 5,4 g rohes 2-Amino-5-nitrobenzthiazol, dessen Identität durch DC-Vergleich mit authentischem Material gesichert wurde.

Beispiel 9

Darstellung von 2-Amino-5-nitrobenzthiazol

Eine Lösung von 6,76 g 2,2'-Diamino-4,4'-dinitrodiphenyldisulfid (Hodgson und Dodgson, J.Chem.Soc. 1948 , 870) und 6,08 g Thioharnstoff in 40 ml Sulfolan wurde 2 Stunden bei 130 °C gerührt. Man verdünnte mit 300 ml Wasser, saugte ab und wusch mit Wasser. Man erhielt 7,2 g rohes 2-Amino-5-nitrobenzthiazol, dessen Identität durch DC-Vergleich mit authentischem Material gesichert wurde.

Beispiel 10

Darstellung von 2-Methyl-5-nitrobenzthiazol

Eine Suspension von 10,13 g 2,4-Dinitro-chlorbenzol und 15 g Thioacetamid in 50 ml Sulfolan wurde auf 100 °C erhitzt und 1 Stunde bei dieser Temperatur gerührt. Nach Erkalten wurde geklärt. Das Filtrat wurde mit 200 ml Wasser versetzt. Man saugte ab, wusch mit Wasser und trocknete. Ausbeute 6,02 g dünnschichtchromatographisch einheitliches Material. Umlösen aus Ethanol hebt den Schmelzpunkt auf 133 °C (J.Chem.Soc. Perkin Trans. 1, 1973 , 356-359 gibt Fp. 135-137 °C an). $m_e^+$: 194

Beispiel 11

Darstellung von 2-Amino-5-nitro-7-trifluormethylbenzthiazol

In eine Lösung von 15,2 g Thioharnstoff in 50 ml Sulfolan trägt man bei 100 °C unter Rühren langsam 13,5 g 2-Chlor-3,5-dinitrobenzotrifluorid ein. Man rührt weitere 2,5 Stunden bei 100 °C, saugt nach Erkalten ab und verrührt den trockenen Rückstand mit 50 ml Schwefelkohlenstoff. Man saugt ab und erhält 7,5 g chromatographisch einheitliches Material vom Fp. 303-307 °C (Zers.). Nach Umlösen aus Pyridin/Wasser (3/1) steigt der Schmelzpunkt auf 315-316 °C (Zers) an. $m_e^+$ : 263

Beispiel 12

Darstellung von 2-Amino-5-trifluormethyl-7-nitrobenzthiazol

In eine Lösung von 76 g Thioharnstoff in 250 ml Sulfolan trägt man bei 100 °C unter Rühren langsam

67,5 g 4-Chlor-3,5-dinitrobenzotrifluorid ein. Man rührt eine weitere Stunde bei 100° C. Nach Erkalten saugt man den Nieder schlag ab und trocknet. Man erhält 35,7 g rohes 2-Amino-5-trifluormethyl-7-nitrobenzthiazol vom Fp. 254-256° C (Zers.). Umlösen aus Pyridin/Wasser (3/1) hebt den Schmelzpunkt auf 262-263° C. $m_e^+$ : 263 Aus dem Filtrat des Rohprodukts können durch Zusatz von 500 ml Wasser weitere 28,7 g ausgefällt werden.

Beispiel 13

Darstellung von 2-Amino-5-ethansulfonyl-benzthiazol

Man erwärmt eine Suspension von 12,8 g Ethyl-4-chlor-3-nitrophenyl-sulfon und 15,2 g Thioharnstoff in 50 ml Sulfolan auf 120° C und hält diese Temperatur 1,5 Stunden. Man trägt auf 600 ml Wasser aus, saugt ab und wäscht mit Wasser. Man erhält 10,6 g farbloses 2-Amino-5-ethansulfonyl-benzthiazol. $\lambda_{max}$ = 234 nm ($CH_3CN$). $m_e^+$ : 242

Beispiel 14

Darstellung von 2-Amino-5-benzolsulfonyl-benzthiazol

In eine Lösung von 15,2 g Thioharnstoff in 50 ml Sulfolan trägt man bei 150° C 14,9 g 4-Chlor-3-nitrodiphenylsulfon ein und hält diese Temperatur 1 Stunde. Man trägt auf 500 ml Wasser aus, saugt ab und wäscht mit Wasser. Das trockene Produkt wird mit 50 ml Schwefelkohlenstoff verrührt. Man saugt ab und erhält 15,5 g rohes 2-Amino-5-benzolsulfonyl-benzthiazol, $m_e^+$ : 290, Fp.: 278-280° C Zers. (aus Dimethylformamid/Wasser 1/1): $\lambda_{max}$ = 245 nm ($CH_3CN$)

Beispiel 15

Darstellung von 2-Amino-5-chlormethansulfonyl-benzthiazol

In eine Lösung von 15,2 g Thioharnstoff in 50 ml Sulfolan trägt man bei 150° C 13,83 g Chlormethyl-4-chlor-3-nitrophenyl-sulfon ein und hält diese Temperatur 1 Stunde. Man arbeitet wie in Beispiel 14 auf und erhält 10 g 2-Amino-5-chlormethansulfonylbenzthiazol. $m_e^+$ : 262, $\lambda_{max}$ = 238 nm ($CH_3CN$)

Beispiel 16

Darstellung von 2-Aminobenzthiazol

Eine Suspension von 9,5 g 2,2'-Dinitrodiphenyldisulfid und 15,2 g Thioharnstoff in 50 ml Sulfolan wurde 4 Stunden bei 100° C und weitere 4 Stunden bei 150° C gerührt. Man klärt und trägt das Filtrat in 250 ml Wasser ein. Die zunächst schmierige Fällung wird aus Ethanol umkristallisiert. Man erhält 0,54 g 2-Aminobenzthiazol, dessen Identität durch DC-Vergleich mit authentischem Material gesichert wurde.

## Ansprüche

**1.** Verfahren zur Herstellung von Benzthiazolen der Formel

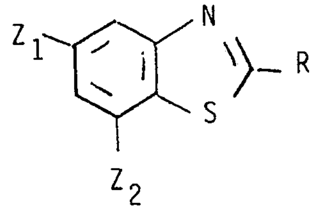

(I)

worin

R für H, $NH_2$ oder $R_1$ steht, wobei

$R_1$ $C_1$-$C_6$-Alkyl oder Phenyl , gegebenenfalls substituiert durch $C_1$-$C_6$-Alkyl, $NO_2$, $CF_3$, -$SO_2$-$C_1$-$C_6$-Alkyl, Halogen oder CN bedeutet,

$Z_1$ und $Z_2$ Wasserstoff, Cl, Br, $NO_2$, $CF_3$, CN, -CO-Alkyl, -$SO_2$-Alkyl, -$SO_2$-Phenyl, -$CO_2$-Alkyl, -CON(Alkyl)$_2$ oder $SO_2N$(Alkyl)$_2$ bedeuten, wobei $Z_1$ und $Z_2$ nicht gleichzeitig für Wasserstoff stehen, die Alkylreste 1-6 C-Atome aufweisen und der Phenylrest durch Cl, Br, $NO_2$ oder $C_1$-$C_4$-Alkoxy substituiert sein kann,

dadurch gekennzeichnet, daß man Verbindungen der Formel

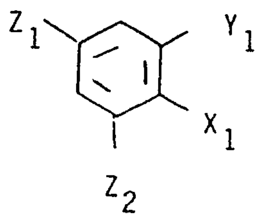

(II)

worin

$X_1$ Chlor, Brom, SH, SCN oder einen Rest der Formel (III)

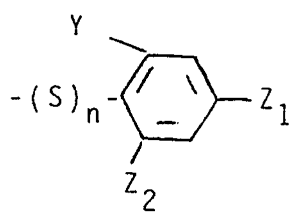

(III)

n = 1 oder 2,

Y $NO_2$ oder $NH_2$,

$Y_1$ $NO_2$ und - falls $X_1$ = SH oder einen Rest der Formel (IIIa) mit n = 2 - auch $NH_2$ bedeuten,

a) mit einer Verbindung, die in einer ihrer möglichen tautomeren Formen der Formel

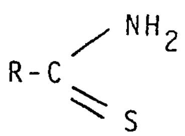

(IV)

entspricht,
oder

b) mit einem Rhodanid der Formel $NH_4{}^+SCN^-$ oder $R_1$-$NH_3{}^+SCN^-$
oder

c) mit einem Gemisch aus Nitrilen der Formel $R_1$-CN oder $NH_2CN$ und $H_2S$,
umsetzt, wobei im Falle der Nitrobenzole ein Überschuß an Thioamid von mindestens 5 - 10 % eines Äquivalents eingesetzt wird.

**2.** Verfahren nach Anspruch 1, dadurch gekennzeichnet, daß man als Verbindung der Formel II solche der Formel IIa

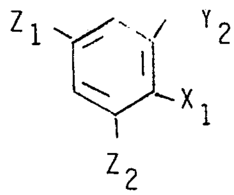

einsetzt, worin

- $Z_1$ $CF_3$, $SO_2CH_3$, $SO_2C_2H_5$, $SO_2C_6H_5$ und $NO_2$,
- $Z_2$ H, $CF_3$ oder $NO_2$,
- X Cl und
- $Y_1$ $NO_2$ bedeuten.

**3.** Verfahren nach Anspruch 1 dadurch gekennzeichnet, daß man als Verbindung der Formel (IV) Thioharnstoff verwendet.

## Claims

**1.** Process for the preparation of benzothiazoles of the formula

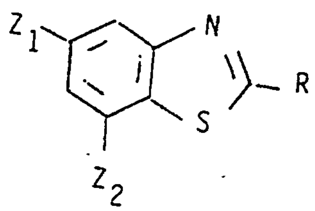

(I)

in which

R represents H, $NH_2$ or $R_1$, $R_1$ denoting $C_1$-$C_6$-alkyl or phenyl, optionally substituted by $C_1$-$C_6$-alkyl, $NO_2$, $CF_3$, -$SO_2$-$C_1$-$C_6$-alkyl, halogen or CN,

$Z_1$ and $Z_2$ denote hydrogen, Cl, Br, $NO_2$, $CF_3$, CN, -CO-alkyl, -$SO_2$-alkyl, -$SO_2$-phenyl, -$CO_2$-alkyl, -CON(alkyl)$_2$ or -$SO_2$N(alkyl)$_2$, $Z_1$ and $Z_2$ not both representing hydrogen, the alkyl radicals having 1-6 C atoms and it being possible for the phenyl radical to be substituted by Cl, Br, $NO_2$ or $C_1$-$C_4$-alkoxy,

characterized in that compounds of the formula in which

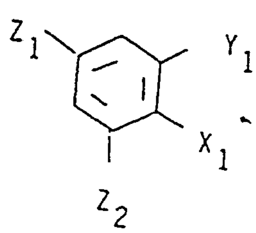

(II)

$X_1$ denotes chlorine, bromine, SH, SCN or a radical of the formula (III)

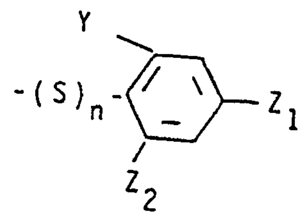

(III)

n = 1 or 2,
y denotes $NO_2$ or $NH_2$, and
$Y_1$ denotes $NO_2$ and, if $X_1$ = SH or a radical of the formula (IIIa) with n = 2, also denotes $NH_2$, are reacted

a) with a compound which, in one of its possible tautomeric forms, corresponds to the formula

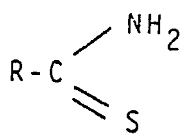

(IV)

or

b) with a thiocyanate of the formula $NH_4{}^+SCN^-$ or $R_1$-$NH_3{}^+SCN^-$

or

c) with a mixture of nitriles of the formula $R_1$-CN or $NH_2CN$ and $H_2S$,

and, in the case of the nitrobenzenes, an excess of thioamide of at least 5-10% of one equivalent is used.

**2.** Process according to Claim 1, characterized in that employed as compound of the formula II is one of the formula IIa

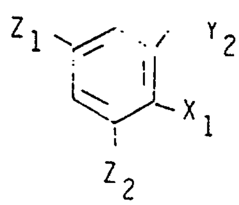

in which

$Z_1$ denotes $CF_3$, $SO_2CH_3$, $SO_2C_2H_5$, $SO_2C_6H_5$ and $NO_2$,
$Z_2$ denotes H, $CF_3$ or $NO_2$,
X denotes Cl, and
$Y_1$ denotes $NO_2$.

**3.** Process according to Claim 1, characterized in that thiourea is used as compound of the formula (IV).

## Revendications

**1.** Procédé de préparation de benzothiazoles de formule

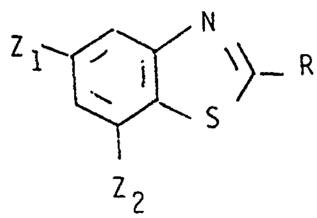

(I)

dans laquelle

R représente H, $NH_2$ ou $R_1$,

$R_1$ est un groupe alkyle en $C_1$ â $C_6$ ou phényle, éventuellement substitué par un radical alkyle en $C_1$ à $C_6$, $NO_2$, $CF_3$, $-SO_2$-(alkyle en $C_1$ a $C_6$), halogéno ou CN,

$Z_1$ et $Z_2$ représentent l'hydrogène, le chlore, le brome, un groupe $NO_2$, $CF_3$, CN, -CO-alkyle, $-SO_2$-alkyle, $-SO_2$-phényle, $-CO_2$-alkyle, -CON-(alkyle)$_2$ ou $-SO_2N$(alkyle)$_2$, $Z_1$ et $Z_2$ ne représentant pas en même temps l'hydrogène, les restes alkyle présentent 1 à 6 atomes de carbone et le reste phényle peut être substitué par Cl, Br, un radical $NO_2$ ou alkoxy en $C_1$ à $C_4$,

caractérisé en ce qu'on fait réagir des composés de formule

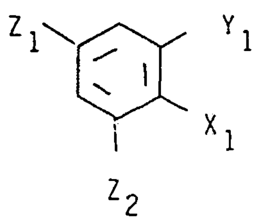

(II)

dans laquelle

$X_1$ représente le chlore, le brome, un groupe SH, SCN ou un reste de formule (III)

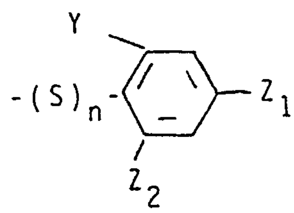

(III)

dans laquelle

n a la valeur 1 ou 2,

Y représente $NO_2$ ou $NH_2$,

$Y_1$ représente $NO_2$ et - au cas où $X_1$ représente SH ou un reste de formule (IIIa) dans laquelle n est égal à 2 - il représente également $NH_2$,

a) avec un composé qui correspond, sous l'une de ses formes tautomères possibles, à la formule

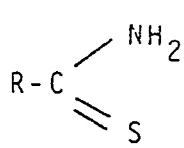

(IV)

ou bien

b) avec un sulfocyanure de formule $NH_4^+SCN^-$ ou $R_1$-$NH_3^+SCN^-$
ou bien
c) avec un mélange de nitriles de formule $R_1$-CN ou $NH_2CN$ et de $H_2S$,
en utilisant dans le cas des nitrobenzènes un excès de thioamide d'au moins 5 à 10% d'un équivalent.

**2.** Procédé suivant la revendication 1, caractérisé en ce qu'on utilise comme composé de formule II un composé de formule IIa

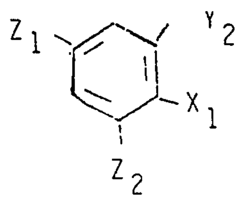

dans laquelle
$Z_1$ représente $CF_3$, $SO_2CH_3$, $SO_2C_2H_5$, $SO_2C_6H_5$ et $NO_2$,
$z_2$ représente H, $CF_3$ ou $NO_2$,
X représente Cl et
$Y_1$ représente $NO_2$.

**3.** Procédé suivant la revendication 1, caractérisé en ce qu'on utilise la thio-urée comme composé de formule (IV).